# EUROPEAN PATENT SPECIFICATION

(11) **EP 2 790 564 B1**
(45) Date of publication and mention of the grant of the patent: **21.01.2026**
(21) Application number: 12858388.7
(22) Date of filing: 13.12.2012
(51) Int. Cl.: A61B 5/145

(54) **DEVICE, SYSTEM AND METHOD FOR IN-VIVO DETECTION OF BLEEDING IN THE GASTROINTESTINAL TRACT**
VORRICHTUNG, SYSTEM UND VERFAHREN FÜR DEN IN-VIVO-NACHWEIS VON BLUTUNGEN IM MAGEN-DARM-TRAKT
DISPOSITIF, SYSTÈME ET PROCÉDÉ POUR LA DÉTECTION IN VIVO DE SAIGNEMENT DANS LE TRACTUS GASTRO-INTESTINAL

(30) Priority: 15.12.2011 US 201161576091 P
(43) Date of publication of application: 22.10.2014
(73) Proprietor: Given Imaging Ltd., 20692 Yoqneam (IL)
(72) Inventor: PASCAL, Amit, 34980 Haifa (IL); GILAD, Zvika, 34987 Haifa (IL); KRUPNIK, Hagai, 36001 Nofit (IL); BIRNBOIM, Yaniv, 44810 Shaarey-Tikva (IL)
(74) Representative: HGF
(86) International application number: PCT/IL2012/050526
(87) International publication number: WO 2013/088444

(56) References cited:
- EP-A1- 2 127 592
- WO-A1-2010/086859
- WO-A1-2010/086859
- JP-A- 2011 036 371
- US-A- 4 567 148
- US-A1- 2004 193 029
- US-A1- 2005 148 842
- US-A1- 2006 036 131
- US-A1- 2008 234 548
- US-A1- 2009 124 874
- US-A1- 2010 010 312

## Description

### FIELD OF THE INVENTION

The present invention relates to the field of in-vivo detection. In particular, the present invention relates to a system for the in-vivo detection of bleeding in the gastrointestinal (GI) tract.

### BACKGROUND OF THE INVENTION

In-vivo bleeding may occur due to different diseases in the body and in various locations along the gastrointestinal (GI) tract. This may indicate different pathologies present at those locations. For example, bleeding in the esophagus may be due to esophagitis or due to ruptures in varices in the esophagus. An ulcer in the stomach, as well as an ulcer in the duodenum, may cause bleeding. And, in the lower digestive tract, colorectal cancer may cause occult bleeding. Therefore, early detection of bleeding along the GI tract may be crucial for better treatment of many patients.

There are some devices and methods, disclosed in the prior art, for detection of bleeding in the GI tract. For example, an endoscope may be used to search for acute bleeding areas in the GI tract. Other devices and methods may involve the patient swallowing dye or radioactive material such that the dye highlights blood vessels within the patient which are then imaged to detect bleeding.

PCT International Patent Application Publication No. WO 2010/086859, assigned to the common assignee of the present invention, described an in-vivo diagnostic device and method for detection of bleeding inside the GI tract. The in-vivo diagnostic device for detection of blood in the GI tract described therein comprises a special housing having one end in the form of a gap through which the in-vivo bodily fluids may flow. Illumination sources, such as LEDs, may reside on one side of the gap and irradiate the bodily fluids passing through the gap. Each illumination source may irradiate the in-vivo fluids at a different narrow band illumination. At least one light detector may be positioned at the opposite side of the gap facing the illumination sources in order to detect light which passes through the in-vivo fluids. The device additionally comprises a transmitter for transmitting detected signals to an external receiver, which is a part of a system for the in-vivo detection of bleeding. The system also includes a processing unit for comparing the detected signals to a predetermined threshold, thereby determining the presence of blood in the GI tract.

However, bile found in the small bowel may have a transmittance spectrum that is similar to the transmittance spectra of blood, such that there may be inaccuracies regarding the determination of the presence of blood in-vivo. For example, if the transmittance spectrum of bile is similar to the transmittance spectra of blood, the device may give an indication as to the presence of blood, when there was actually bile present in the examined area. Therefore, it is very important to determine that the transmittance spectra indicate the presence of blood and not the presence of bile.

The device described in WO 2010/086859 overcomes the deficiencies of the prior art devices by being able to detect the presence of bile in the GI tract. Thus, the device disclosed in WO 2010/086859 is able to determine whether the absorption or transmittance spectra measured by the light detector in-vivo indicates the presence of blood, the presence of bile or the presence of both. Furthermore, the system disclosed in WO 2010/086859 is able to determine the concentration of both bile and blood found in-vivo.

As mentioned in WO 2010/086859, the sensing head of the in-vivo device comprises several, for example, four, different LEDs. Two of the LEDs irradiate at two different wavelengths selected from the range between 400nm to 650nm, for example 560nm and 610nm, while the other two LEDs irradiate at two different wavelengths selected from the range of 650nm to 900nm, for example 700nm and 800nm. The defined blood score may be calculated from the ratio of detected light that is irradiated by one LED at 560nm and another LED irradiating at 610nm, whereas the defined bile score may be calculated from the ratio of detected light that is irradiated by one LED at 700nm and another LED at 800nm. If the ratio from the readings of 560nm and 610nm is zero, i.e., the spectra is a flat line, it may be concluded that the irradiated fluid flowing through the sensing head gap contains no blood. If the ratio from the readings of 700nm and 800nm is zero, i.e., the spectra is a flat line, it may be concluded that the irradiated fluid contains no bile.

However, there are several deficiencies in the aforementioned device disclosed in WO 2010/086859, for example a limited number of LEDs (preferably four) that does not allow differentiating between blood and other materials found in the bodily fluid of the GI tract, such as chlorophyll having the same absorption band between 600nm and 700nm as blood.

It has recently been found that there is also a problem of a relatively wide irradiation angle of LEDs located on the sensing head. In this case, light from such LEDs might strike objects (especially tissue) and change the reading in the photodiode.

It would be also beneficial to add temporal information to the system in order to identify active bleeding that occurred in the near past and to present a time line of the bleeding event. In other words, memory capability in the in-vivo diagnostic device and system are highly desirable.

### SUMMARY OF THE INVENTION

The aforementioned deficiencies of the in-vivo diagnostic device and system described in WO 2010/086859 may be overcome by using an improved diagnostic device and system for the in-vivo detection of bleeding in the GI tract. It is, therefore, an object of the present invention to provide an improved system for the in-vivo detection of bleeding in the GI tract.

According to the present invention, there is provided an in-vivo diagnostic system for the detection of bleeding inside the GI tract of a patient, as defined in claim 1 of the appended claims.

In one embodiment, the in-vivo diagnostic device may be an autonomous swallowable non-imaging capsule. The device is assembled inside a case (hereinafter the "housing"), which may have a substantially transparent portion.

The housing comprises a sensing head in the form of a gap, which constantly remains in contact with bodily fluids and through which the fluids may constantly pass or flow.

The sensing head comprises at least three LEDs positioned on a first side of the gap, and at least one light detector photodiode positioned on a second side of the gap opposite to the first side and facing the LEDs. There may be at least six illumination sources, e.g., LEDs, which may be positioned on one side of the gap, irradiating at different narrow band wavelengths, while at least one light detector photodiode is positioned on the opposite side of the gap. The light detector photodiode is positioned such that it is facing the illuminating LEDs, while the gap is situated between the LEDs and the light detector photodiode. As the fluids pass through the gap, light irradiated by the LEDs passes through the bodily fluids and onto the light detector photodiode. Some of the light may be absorbed by the fluids, some may be scattered by insoluble particles, some may be reflected, and some may be transmitted to the light detector photodiode, which then transmits signals, created in response to the detected light, to an external receiver.

A processor, external to the device, processes the signal sent by the light detector and may create an absorption or transmittance spectra of the bodily fluids. By comparing the signals to a reference transmittance spectrum of other materials typically present in the GI tract, such as bile and chlorophyll, and to a reference transmittance spectrum of blood, it may be determined whether bile, blood, chlorophyll or all are present in the bodily fluids, and in what concentration, such that a conclusion may be made regarding presence of in-vivo pathologies.

In some embodiments, instead of comparing transmittance or absorbance spectra, a comparison between discrete signals detected by the light detector photodiode and a predetermined threshold may be made.

In yet a further embodiment, the sensing head of the in-vivo diagnostic device may include a specific blocker surrounding the illumination sources on one side of the gap thus reducing external reflection.

The internal components of the in-vivo diagnostic device may reside on a stepped printed circuit board (PCB). The PCB may optionally include one or more components, e.g., conductive rings. Other designs, components, elements, and structures may be used in addition to and/or in place of the rings, steps, etc. The PCB may further include contact points to connect additional components.

In yet a further embodiment, the PCB may include a sensor for sensing the current location of the device and an antenna typically associated with a transmitter for transmitting data from the device to an external system, which may determine the location of the device in a segment resolution. For example, the system may determine the location of the device along the GI tract segments, e.g., determine whether the device is in the esophagus, the stomach, the small bowel or the colon, based on the presence and/or concentration of bile. Other methods may be used to determine the location of the device in the different organs along the GI tract.

In some embodiments, other localization methods may be used for determining where the in-vivo device is located in-vivo along the GI tract. For example, the in-vivo device may include a pH detector which may be built into the sensing head and which may continuously detect pH levels of the body fluids inside the GI tract. This pH detector may comprise two electrodes and an electrical circuit, and may transmit the detected pH to a receiver that is external to the patient's body. Since, in different areas of the GI tract, there are different pH levels, the detected pH level may indicate the location of various pathological lesions in the GI tract. Thus, in a particular embodiment, the in-vivo diagnostic device may detect both the absorption or transmittance spectra and the pH level.

In another embodiment, the in-vivo diagnostic device may contain an imager in order to acquire in-vivo images of the pathological lesions where the bleeding may occur. The in-vivo device may also contain a power source, such as batteries.

In some embodiments, the in-vivo diagnostic device may additionally include therapeutic means. In this case, it would be desirable to have full control over the movement of such in-vivo diagnostic device including steering and maneuvering this device to a desired location and orientation of the device in the GI tract. This fully maneuverable in-vivo device may detect and immediately treat the bleeding lesions during its passage through the GI tract. The in-vivo diagnostic device may include a permanent magnet assembly for interacting with external magnetic fields for generating forces for steering the device. In addition, this in-vivo diagnostic device may include a multilayered imaging and sensing printed circuit board for sensing the current location and orientation of the in-vivo device, and for transmitting corresponding location and orientation data to an external system that generates the external magnetic fields.

The system of the invention includes the in-vivo diagnostic device, an external receiver/recorder able to receive data (e.g., absorbance or transmittance values) transmitted by the in-vivo device, and may include a computing platform or workstation able to store, process, display, or analyze the received data.

The in-vivo diagnostic system of the invention may perform a method of in-vivo diagnostics. The method for detecting blood in-vivo may include the following steps:
(i) swallowing the in-vivo diagnostic device of the present invention;
(ii) in-vivo irradiating of the bodily fluids at different wavelengths as the device moves along the GI tract;
(iii) detecting over time the absorbance or transmittance signals or measuring absorbance or transmittance spectra of the in-vivo bodily fluids that pass through the sensing head of the device;
(iv) processing the absorbance or transmittance signals or spectra over time; and
(v) displaying the processed absorbance or transmittance signals or transmittance spectra as a function of time.

The method may additionally include comparing the processed absorbance or transmittance signals or transmittance spectra with a predetermined threshold. The method may further include comparing the absorbance or transmittance spectra of the in-vivo bodily fluids to a predetermined absorbance or transmittance spectra of bile and chlorophyll and determining the presence and concentration of bile and chlorophyll in the bodily fluids.

The method may further include the step of determining concentration of blood in the bodily fluids over time, following the step of processing the detected absorbance or transmittance signals or spectra over time. The method may include the step of displaying the processed absorbance or transmittance signals or spectra over time, following the step of processing the detected absorbance or transmittance signals or spectra over time. Displaying the processed absorbance or transmittance signals or spectra over time may include displaying concentration of blood over time. The step of displaying may include displaying concentration of blood, alongside displaying a video of in-vivo images stream over time.

The method may further optionally include acquiring an in-vivo image or other data of the GI tract (other than absorbance or transmittance signals or spectra), such as pH, transmitting the acquired in vivo-image or other data, analyzing the in-vivo image or other data, and/or other suitable operations.

In order to be able to display data related to bleeding profiles or bleeding events over time, the external receiver and optionally the in-vivo diagnostic device includes a memory. The data collected over time by the sensing head (and, in some embodiments, the imaging data) is stored in the memory unit located in the external receiver or and optionally in the in-vivo device. The stored data may then be processed and analyzed by, for example, a computing platform, which may be operatively associated with the receiver or the device. The processed data (e.g., blood concentration over time) may then be displayed to the physician so the physician could make a quick and precise diagnosis on patient condition.

In other embodiments, the external receiver comprises the memory unit, the processing unit and a display, such that there is no need to transfer the collected data to an additional computing platform. In these embodiments the entire system may comprise the in-vivo diagnostic device and the external receiver alone, which may make it easier on a patient to undergo the majority, if not the entirety, of the procedure of blood detection in a home setting, instead of in a hospital setting.

Various embodiments of the invention may allow various benefits, and may be used in conjunction with various applications. The details of one or more embodiments are set forth in the accompanying figures and the description below. Other features, objects and advantages of the described techniques will be apparent from the description and drawings and from the claims.

### BRIEF DESCRIPTION OF THE DRAWINGS

The present invention will be understood and appreciated more fully from the following detailed description taken in conjunction with the appended drawings in which:
Figs. 1A-1B are cross-sectional front view and side view of a prior-art non-imaging in-vivo diagnostic device, respectively;
Fig. 2 is a transmittance spectrum of the three major components absorbing in the bodily fluid of the GI tract between 500nm to 800nm;
Fig. 3 is an emission spectrum of the six experimentally chosen LEDs for the in-vivo diagnostic device, in accordance with embodiments of the present invention;
Fig. 4A is a front view of an imaging in-vivo diagnostic device, constructed and operative, in accordance with embodiments of the present invention;
Fig. 4B is a cross-sectional front view of an imaging in-vivo diagnostic device, constructed and operative, in accordance with embodiments of the present invention;
Fig. 4C is a cross-sectional side view of an imaging in-vivo diagnostic device, constructed and operative, in accordance with embodiments of the present invention;
Fig. 5A is a perspective view of the printed circuit board assembly (face side), in accordance with embodiments of the present invention;
Fig. 5B is a perspective view of the printed circuit board assembly (back side), in accordance with embodiments of the present invention;
Fig. 5C is a perspective view of the folded printed circuit board assembly, in accordance with embodiments of the present invention;
Figs. 6A-6B are front views of different imaging in-vivo diagnostic devices, in accordance with embodiments of the present invention;
Figs. 6C-6D are front and side views of an imaging in-vivo diagnostic device, respectively, in accordance with the present invention;
Fig. 7 is a schematic presentation of the system for the in-vivo detection of bleeding in the GI tract, in accordance with embodiments of the present invention;
Fig. 8 is a graph describing different bleeding profiles in accordance with embodiments of the present invention;
Figs. 9A-9B are graphs showing detected light signals as a function of time before and after processing, respectively.
Fig. 10 is a flow chart describing a method of using an in-vivo diagnostic device in accordance with embodiments of the present invention; and
Fig. 11 is a flow chart describing a method of using an in-vivo diagnostic device for detection of bleeding in the GI tract, in accordance with other embodiments of the present invention.

It will be appreciated that, for simplicity and clarity of illustration, elements shown in the figures have not necessarily been drawn accurately or to scale. For example, the dimensions of some of the elements may be exaggerated relative to other elements for clarity, or several physical components may be included in one functional block or element. Further, where considered appropriate, reference numerals may be repeated among the figures to indicate corresponding or analogous elements.

### DETAILED DESCRIPTION OF THE INVENTION

In the following description, various aspects of the invention will be described. For purposes of explanation, specific configurations and details are set forth in order to provide a thorough understanding of the invention. However, it will also be apparent to one skilled in the art that the invention may be practiced without the specific details presented herein. Furthermore, well-known features may be omitted or simplified in order not to obscure the invention.

Some embodiments of the invention may be used, for example, in conjunction with in-vivo sensing of pH, temperature, pressure and/or electrical impedance, in-vivo detection of a substance or a material, in-vivo diagnostics and imaging of a medical condition or a pathology, in-vivo acquisition or analysis of data, in-vivo therapy and various other in-vivo sensing, imaging and therapeutic devices, systems, and methods.

The in-vivo diagnostic device of one embodiment may typically be fully autonomous and typically self-contained. For example, a device according to some embodiments may be a capsule or other unit where all the components are substantially contained within a case (hereinafter the "housing"), and where the device does not require wires or cables in order to receive power or transmit information.

According to a particular embodiment, the in vivo device is essentially floatable or has a neutral or near neutral buoyancy in water or in other liquids that may fill body lumens of the GI tract. Accordingly, the device may have a specific gravity of 1 in reference to water or it may have a specific gravity of about 1 in reference to water. The in-vivo diagnostic device according to one embodiment may be designed to access pathologic lesions in nearly every region of the gastrointestinal (GI) tract, including the colon and biliary tree. In some embodiments, the in-vivo device may be designed to collect data only in the pathological areas and to bypass the healthy areas, and it may be designed to access difficult to reach areas, where tethered endoscopes cannot reach or cannot reach easily.

Some embodiments are directed to a typically swallowable in-vivo diagnostic device in a form of a swallowable capsule that may be used for diagnosing the pathological areas inside the GI tract where bleeding occurs.

In addition, components of the device, according to embodiments of the invention, may be similar to components used in the PillCam^{®} capsule endoscopy system commercially available from Given Imaging Ltd., Yoqneam, Israel, which is the assignee of the present invention. Of course, devices, systems, structures, functionalities and methods as described herein may have other configurations, sets of components and processes, etc.

It should be also noted that, while the system of the present invention may be used, for example, in a human body, the invention is not limited in this respect. For example, some embodiments may be used in conjunction with or inserted into a non-human body, e.g., a dog, a cat, a rat, a cow, or other animals, pets, laboratory animals, etc.

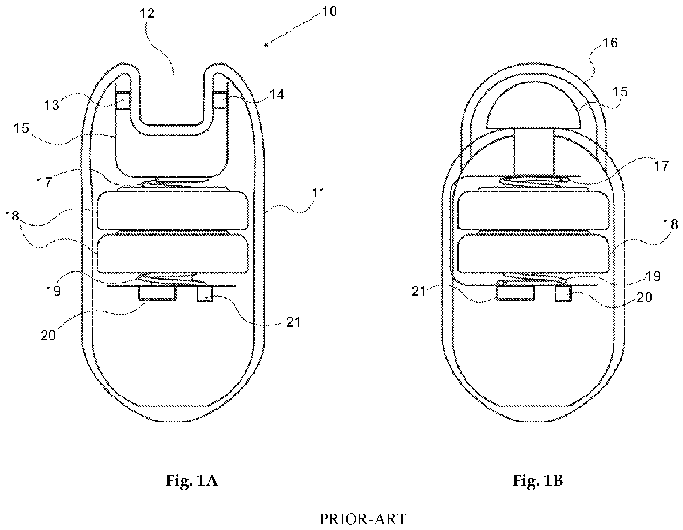

As mentioned above, the in-vivo diagnostic device, according to one embodiment, is based on the in-vivo device described in WO 2010/086859 and shown in the prior-art Figs. 1A and 1B, which demonstrate a cross-sectional front and side view of a non-imaging in-vivo diagnostic device for the in-vivo detection of bleeding, respectively. This prior-art device comprises housing 11. One end of the housing forms gap 12, which may be hydrodynamically curved in order to allow continuous flow of the bodily fluids through it. In some embodiments, the width of gap 12 may be between 4-5 mm, although other widths may be used. In order for gap 12 to allow continuous flow of the fluids through it, the in-vivo device should constantly be in contact with the body fluids in the GI tract. Therefore, in some embodiments, the device has a specific gravity of just above 1. When the specific gravity of the device is above 1, the device may pass through the colon in an optimal way. Specific gravity of just above 1 may ensure, on one hand, that the in-vivo diagnostic device does not float above the fluids, i.e., that the device and, more important, gap 12 is in constant contact with the fluids, and, on the other hand, may ensure that the device does not sink to the bottom of the body lumen wall in the GI tract and, consequently, lose its ability to move freely.

In some embodiments, in order to avoid entry of big insoluble particles found in the GI tract into gap 12, thereby perhaps blocking it, gap 12 may include a membrane cover or a hydrogel cover (not shown). The membrane or hydrogel may cover the entire gap 12, and may have holes or pores that allow only particles of a certain size or smaller to pass through them. On the other hand, the holes or pores in the membrane should not be so small so as to prevent the constant flow or passage of the liquid (due to surface tension) and blood particles through the gap. For example, the size of the holes or pores may be about 0.1-1 mm.

In a further embodiment, housing 11 of the in-vivo diagnostic device may be made transparent or semi-transparent, and of any suitable biocompatible material, for example, Parylene^{®} , Parylene C^{®} , Isoplast^{®} or Makrolon^{®} . Other biocompatible materials may be used.

In one embodiment, there are at least four illumination sources, e.g., LEDs 13, located on one side of the gap 12, irradiating at different wavelengths, while there is at least one light detector photodiode 14 located on the opposite side of the gap 12.

In one embodiment, light detector photodiode 14 is positioned such that it is directly facing LEDs 13, while gap 12 is located between LEDs 13 and photodiode 14. Light irradiated by LEDs 13 passes through the bodily fluids and strikes photodiode 14. Some of the light may be absorbed by the fluids, some may be scattered by insoluble particles, some may be reflected, and some may be transmitted to photodiode 14, which then transmits signals, created in response to the detected light, to an external receiver. According to some embodiments, LEDs 13 may irradiate at a low frequency in order to save energy during the procedure of blood detection. Photodiode 14 may also be activated in synchronization with LEDs 13, e.g., the signals from the in-vivo device may be detected every 10 sec or every 1 minute. Other frequencies may be used.

The non-imaging in-vivo diagnostic device shown on Figs. 1A-1B may comprise a printed circuit board assembly (PCB) 15 onto which LEDs 13 and photodiode 14 are electronically connected. The PCB 15 may be made of rigid portions and flexible portions. Onto PCB 15 may further be mounted a transmitter 20 and antenna 21.

The photodiode 14 may pass to the transmitter a signal created by the detected light, which had passed through the in-vivo fluids. In order to preserve energy, transmitter 20 may be synchronized with the light detector 14. The device may further comprise a power source 18, such as silver-oxide batteries, and power source contacts 17 and 19 which are both mounted on PCB 15. Power source 18 should supply enough power to keep the device operating during its passage through the entire GI tract, e.g., at least for as long as 72 hours.

The power source 18 may take the form of internal batteries, power cells, or power circuitry such as a wireless power receiving unit based on RF power transmission, which may be included in the device. The battery within the power source 18 may be very small. An example of a suitable battery is a silver oxide battery often used to power watches, lithium batteries or any other suitable electrochemical cells having a high energy density. The model battery has voltage of 1.55 volts and a capacity of 12.5 mA-hours and has a disk-like shape with a diameter of approximately 5.7 mm and a thickness of approximately 1.65 mm. With a typical range of power requirements, the model battery can be expected to power the in vivo device for between approximately two weeks and eighteen months, depending on actual usage conditions. Other suitable power sources may be used. For example, power source 18 may receive power or energy from an external power source (e.g., an electromagnetic field generator), which may be used to transmit power or energy to the in-vivo diagnostic device.

In further embodiments, power source 18 may be rechargeable via induction or ultrasonic energy transmission, and may include an appropriate circuit for recovering transcutaneously received energy. For example, power source 18 may include a secondary coil and a rectifier circuit for inductive energy transfer. In still other embodiments, power source 18 may not include any storage element, and thein-vivo device may be fully powered via transcutaneous inductive energy transfer. As an example, such power source is commercially available from Medtronic, Inc. of Minneapolis, Minn.

The LEDs 13 may operate in an alternating or sequential mode with different pulse duration, in order to distinguish between the different LEDs being constantly detected by the photodiode 14. For example, one LED 13 may irradiate the bodily fluids in the GI tract for a certain predetermined time period and then stop, and a second LED 13 may begin irradiating the bodily fluids for another time period. When the second LED stops irradiating, the third LED may begin irradiating for yet another predetermined time period, and so on.

In some embodiments, the predetermined duration of irradiation may differ for each LED, but in other embodiments, they may all irradiate for the same duration, one subsequent to the other. The photodiode 14 may then detect light, which passes through the bodily fluids, from one of the LEDs 13 at a time.

One of the problems solved by the present invention is differentiating between blood and other components of the bodily fluid in the GI tract, while having a limited number of LEDs. For example, one may differentiate bile from blood by measuring the absorption above 600nm. Thus, the intensity gradient in the absorbance area of 600-700nm is indicative of the bile presence, whereas the intensity gradient in the absorbance area of 400-600nm is indicative of the blood presence.

In a particular embodiment, there may be at least six LEDs, preferably white with different filters for illuminating at a specific narrow wavelength. The selection of the LEDs irradiating at different specific wavelengths is not a trivial task and constitutes an essential part of the present invention.

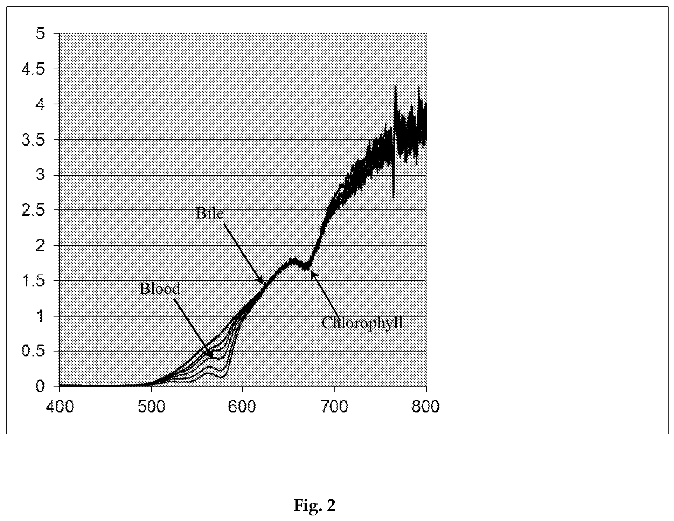

Reference is now made to Fig. 2, which shows absorption spectra of the three major components absorbing in the bodily fluid of the GI tract between 500nm to 800nm. Spectroscopic measurements of the human colon samples in the absorption range of 500-800nm surprisingly showed some additional shoulders in the blood absorbance/transmittance spectrum, which cannot be attributed to the spectrum of blood. After evaluation of the results, two other components (in addition to blood), contributing to the overall absorbance spectrum of the bodily fluid from the GI tract, were determined to be bile and chlorophyll.

Since the absorbance spectrum of chlorophyll in water is known, it is possible to normalize the measured absorption during the in-vivo detection of blood in the GI tract, and to eliminate the contribution of chlorophyll in the transmitted absorbance data. This normalization may be done similarly to how the contribution of bile is eliminated from the transmitted absorbance data of blood, as described in WO 2010/086859.

Based on a new and unique algorithm developed by the common assignees of the present application and described elsewhere, at least six LEDs have been selected to account for the absorbance of blood, bile and chlorophyll in the bodily fluid of the GI tract. For example, chlorophyll has an absorbance peak at 670nm, so the in-vivo diagnostic device should include the LEDs irradiating at approximately that wavelength, as well as the LEDs irradiating in range of 610-620nm, which would help to determine the slope of the absorbance band.

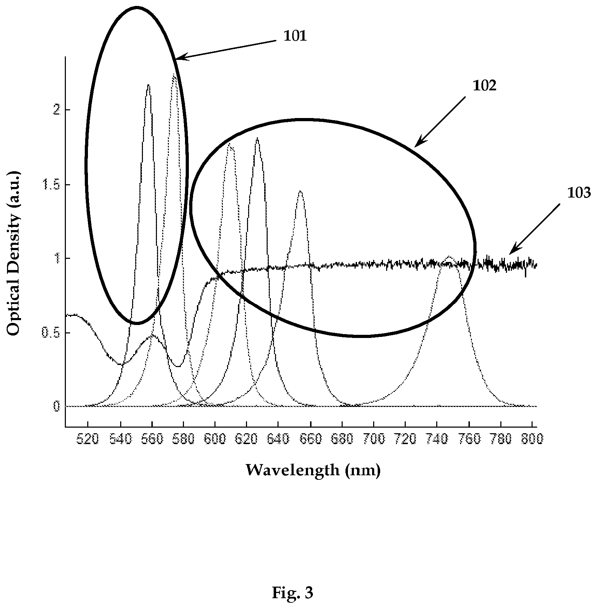

Reference is now made to Fig. 3 showing the emission spectra of the six experimentally chosen LEDs, in accordance with embodiments of the present invention, though other LEDs may be chosen. The configuration of LEDs was selected out of several configurations that gave similar results on the limited dataset. For example, as shown on Fig. 3, the first two LEDs emitting at 557nm and 574nm irradiate at the absorbance area of blood (101). Other four LEDs emitting at 609, 626, 653 and 747nm irradiate in the area where the absorbance of blood is insignificant but where bile and chlorophyll do absorb well (102). Typical blood absorbance spectrum is represented by line 103. The LEDs in the area 102 are selected away from the blood steep slope in order to reduce sensitivity of the signal. In addition, the commercial LEDs drift and spectra overlap of about 10nm is taken into account.

Typical LEDs 13 have a relatively wide irradiation angle. Light from these LEDs might strike objects (e.g. tissue or the edges of the housing of device 10) and change the reading in the photodiode 14. In order to overcome this problem, in a particular embodiment, a blocker (not shown), which is similar to a fence surrounding the LEDs 13 area, and slightly higher than these LEDs, is placed around them. In some embodiments, the blocker may have a dark opaque color in order to block passage of light through it.

The ranges which LEDs 13 may be selected from are separated into three; the first range is between 400nm and 600nm, where blood absorbance is significant, the second range is between 600nm and 700nm, where absorbance of bile and chlorophyll is significant whereas absorbance of blood is insignificant, and the third range is between 700nm and 900nm, which is used for normalizing the absorbance of floaters floating in-vivo. At least one LED is selected from each of the three ranges mentioned above. According to the example provided above, LEDs emitting at 557nm and 574nm are selected from the first range, LEDs emitting at 609, 626, and 653nm are selected from the second range, and an LED emitting at 747nm is selected from the third range. Other LEDs may be selected out of each of the three ranges.

In some embodiments, as described above, device 10 may comprise six LEDs 13. In these embodiments, the six LEDs 13 may irradiate at approximately 560, 575, 610, 625, 650 and 750nm. For example, approximately may mean the LEDs 13 may irradiate at the wavelengths mentioned plus minus 5nm, though other deviations may be used. In the example above, the six LEDs 13 emit light at wavelengths 557, 574, 609, 626, 653 and 747nm, all of which conform to the 5nm deviation, though other deviations may be used.

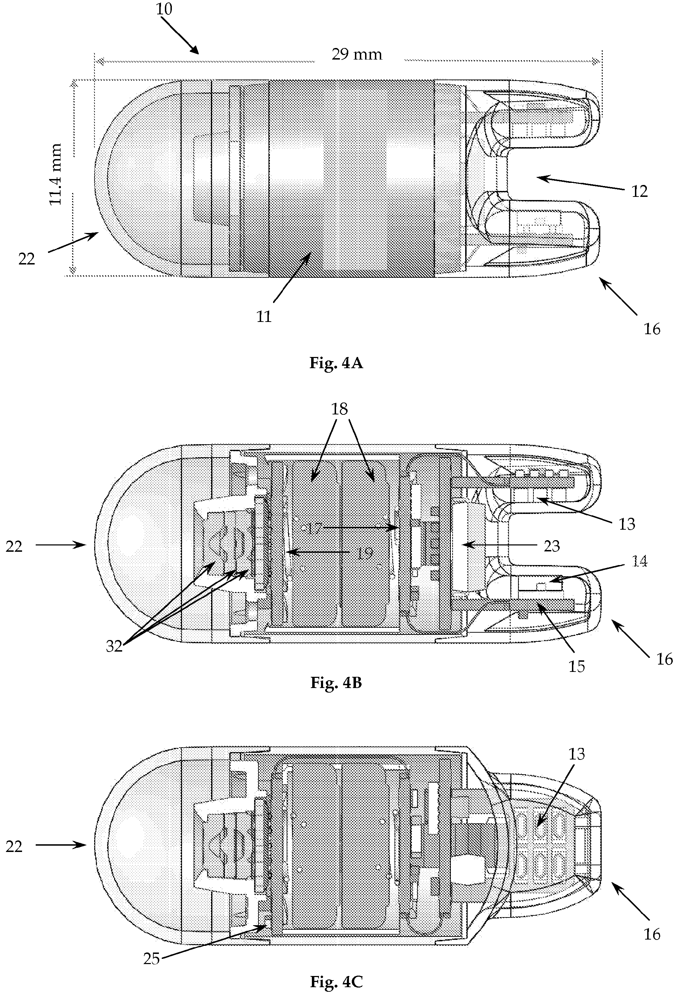

Another LED that may be selected for use in device 10 (Figs. 4A-4C, described in detail below) may be an LED emitting at 660nm, since chlorophyll b has high absorbance at 660nm. This LED may either be used in addition to the list of six LEDs provided above, or it may replace one of the LEDs listed above, for example, LED emitting at 660nm may replace the LED emitting at 653nm.

The LEDs 13 may be any commercially available LEDs, such as Hyper TOPLED^{®} by Osram^{™}, KPHHS-1005SECK^{®} or KPHHS-1005CGCK by Kingbright^{™}, ED-109UYGL, ED-107UR or ED-012UORS by Optotech, ELC-740-25 by Epigap, or TLPGE1008A by Toshiba.

The photodiode 14 may be, for example, OPT101^{®} by Burr-Brown Products^{™} from Texas Instruments, MLX75305C^{®} by Melexis^{™} Microelectronic Integrated Systems, or TSL12S-E23^{®} , TSL13T, or TSL257T by TAOS^{™} (Texas Advanced Optoelectronic Solutions).

In one embodiment, the in-vivo diagnostic device may also be an imaging device, e.g., it may contain an imager in order to acquire in-vivo images of the pathological lesions in the GI tract where the bleeding may occur. Reference is now made to Figs. 4A, 4B and 4C, which show a front view, a cross-sectional front view and a cross-sectional side view of an imaging in-vivo diagnostic device 10, constructed and operative, in accordance with embodiments of the present invention, respectively. This imaging in-vivo device may be based on the PillCam^{®} capsule endoscopy system commercially available from the common assignee of the present invention.

The components of the imaging in-vivo diagnostic device 10 may be similar to the components of the non-imaging in-vivo device, as described above. Housing 11 may form gap 12, which may be hydrodynamically curved in order to allow continuous flow or passage of the bodily fluids through it. In some embodiments, the width of gap 12 may be between 4-5 mm, although other widths may be used. Illumination sources, e.g., LEDs 13 are placed on one side of the gap 12, each irradiating at a different narrow band wavelength, while on the opposite side of the gap there is at least one light detector or photodiode 14, which is positioned such that it is directly facing LEDs 13. In some embodiments, the number of LEDs 13 may be six, while in other embodiments other numbers may be used. The end of device 10, which comprises gap 12, LEDs 13, and light detector or photodiode 14 forms sensing head 16.

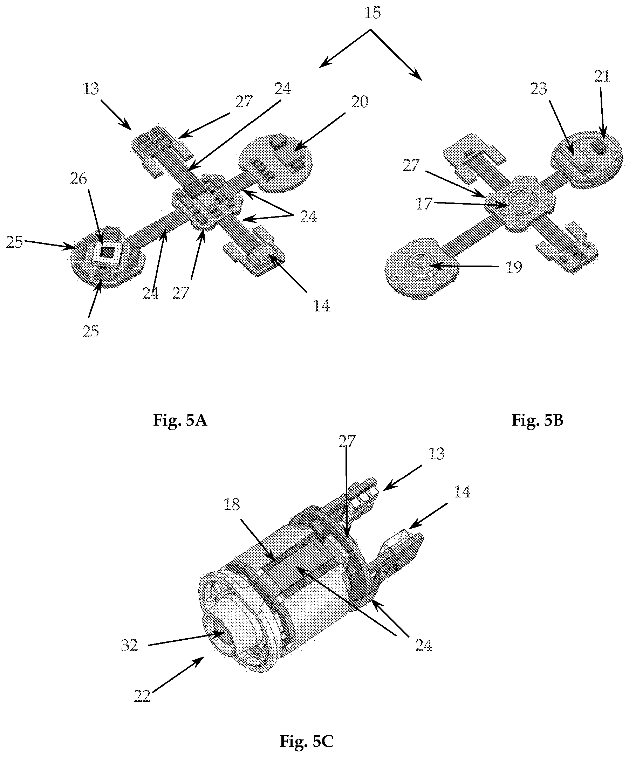

Reference is now made to Figs. 5A and 5B showing printed circuit board assembly (PCB) 15 that may have a rigid portion, onto which LEDs 13 and photodiode 14 are oppositely mounted, so that photodiode 14 would directly faces LEDs 13. In one embodiment, the shape of the rigid portion of PCB 15, onto which LEDs 13 and photodiode 14 are mounted, may be any shape, as long as it complies with the shape and size of both sides of the in-vivo device, on either side of gap 12.

Typically, the contour of the device should be round with no sharp edges, so that it is suitable for in-vivo insertion either by swallowing or through other methods, such that it would not cause any damage to tissue during insertion. Furthermore, the device should be designed with rounded edges so it does not cause any harm to tissue while passing along the GI tract by natural peristalsis. Thus, the shape of the rigid potion of PCB 15, to which LEDs 13 and photodiode 14 are connected, as shown in Figs. 5A and 5B, may be suitable. In other embodiments, other shapes such as a triangle, rectangle, and square may be used for PCB 15, as long as the shape of housing 11 of the in-vivo diagnostic device covering PCB 15 is not sharp and is suitable for insertion in-vivo, since that is the part that actually comes in contact with the in-vivo tissues.

In a further embodiment, flexible portions 24 of PCB 15 may be folded in order to adjust the shape of the PCB to fit within the volume of the in-vivo device. For example, as shown on Figs. 5A and 5B, flexible portions 24 connect the five rigid portions to one central rigid portion. The flexible portions 24 are then folded, as shown on Fig. 5C to create a U shape which fits the U shape of the device, designed such that there is room between the rigid portions in order to form gap 12. Connected to the rigid portion may be battery contacts 17 and 19. In between battery contacts 17 and 19, batteries 18 may be inserted, as shown in Figs. 4B and 4C.

In another embodiment, the device may include imaging head 22, as shown in Fig. 4A-4C and 5C, in order to acquire in-vivo images of a body lumen. In this case, the combined mode of operation of the two heads (sensing head 16 and imaging head 22) may be either simultaneous, for example when the device is in the stomach or small bowel, or only sensing head 16 may operate when the device is in the colon (thus saving energy). In other embodiments, the sensing head and the imaging head may operate simultaneously throughout the entire GI tract.

Imaging head 22 of the device 10, as shown in Figs. 4B, 4C and 5C, may include one or more illumination sources 25, such as LEDs or other suitable illumination sources, and lenses 32 placed behind a transparent convex (e.g., dome-shaped) optical window.

In a particular embodiment, imaging head 22 of the in-vivo device may include an in-vivo camera/imager 26, as shown on Fig. 5 A. The LEDs 25 may, for example, illuminate a body lumen or cavity being diagnosed. An imaging optical system, may comprise, for example, one or more optical elements, such as one or more lenses 32 or composite lens assemblies, one or more suitable optical filters, or any other suitable optical elements, and may be included in the in-vivo device and may aid in focusing reflected light onto an imager (not shown), focusing illuminated light, and/or performing other light processing operations.

In one embodiment, the device may contain reed switch 23, which may be actuated by magnets and used as a proximity sensor. It may also be used to switch off the device when the operation is not needed, in order to extend the battery life. In a further embodiment, PCB 15 may comprise a transmitter 20 and antenna circuit 21, which are shown on Figs 1A-1B and 5A-5B.

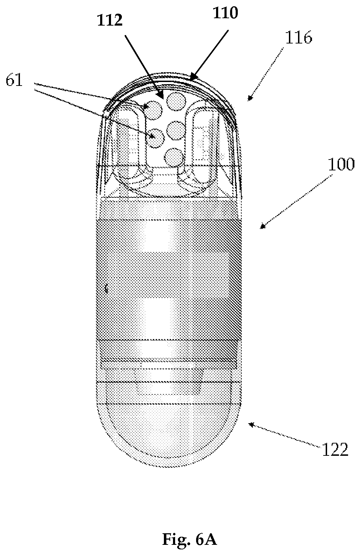

Reference is now made to Figs. 6A-6C which illustrate front views of different imaging in-vivo diagnostic devices, in accordance with embodiments of the present invention. According to some embodiments, gap 12, through which bodily fluids (carrying or not carrying blood related particles) pass, may be blocked by large particles flowing within the bodily fluids. In some embodiments, air bubbles flowing within the bodily fluids may be caught inside gap 12. In yet other embodiments, the fluids within the GI tract may experience turbulences. When, for example, device 10 passes through the small bowel, there may be turbulences in the bodily fluids that may cause device 10 to be pushed against the tissue wall of the GI tract. The GI tract tissue may then be pushed into gap 12 thus at least partially blocking LEDs 13 from irradiating the bodily fluids. When gap 12 is blocked such that constant flow of bodily fluids through it is prevented, the operation of sensing head 16 may be interfered. In order to overcome possible blockage of gap 12, a few in-vivo devices different than device 10 may be used.

For example, device 100 shown in Fig. 6A may comprise a cover 110 that covers the entire sensing head 116. Cover 110 may be made of, for example, silicon, which is safe to use in-vivo as it is a biocompatible material, though other materials may be used. In some embodiments, cover 110 may be placed over the entire sensing head 116 following assembly of all plastic parts of device 100, which constitute the shell of device 100. Cover 110 may comprise holes 61 located on both sides of gap 112 (which is now covered by cover 110). Holes 61 may be located on the sides of gap 12 that are perpendicular to LEDs 13 and to photodiode 14, and which are located in between the LEDs 13 and photodiode 14. In some embodiments, no holes are located on the top end of gap 112, or the top end of cover 110. The top end of cover 110 (or top end of gap 112) may be on a plane parallel to the plane of rigid portion 27 of PCB 15 (Figs. 5A-5C), and farthest from it around the contour of cover 110. However, in other embodiments, holes similar to holes 61 may be located at the top end of cover 110 in addition to the holes 61 located on the sides of gap 112 (described above). The size of each of holes 61 may be large enough so as to allow free passage of blood particles, and yet small enough to prevent passage of large particles that might block gap 112, thus blockage of irradiation from LEDs 13 towards bodily fluids and/or blockage of absorbance or transmittance signals of the bodily fluids towards photodiode 14 may be avoided. In some embodiments, cover 110 and holes 61 may be replaced with a net or mesh comprising holes at an appropriate size that allow free passage of blood particles but prevent passage of large particles, as described above. In some embodiments, device 100 may comprise an imaging head 122 located opposite the sensing head 116, whereas in other embodiments, device 100 need not comprise an imaging head 122 in addition to sensing head 116.

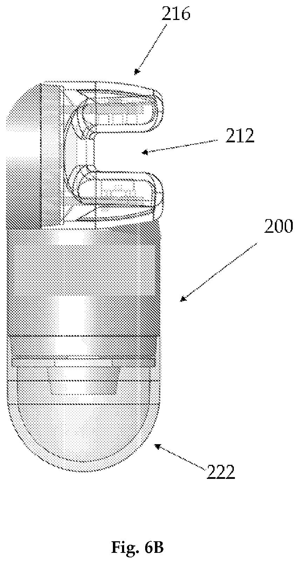

Another example of an imaging in-vivo diagnostic device may be device 200, shown in Fig. 6B. In some embodiments, device 200 may comprise an imaging head 222 located opposite a sensing head 216. Yet, in other embodiments, device 200 need not comprise an imaging head 222 in addition to sensing head 216. In some embodiments, the sensing head 216 may comprise a gap 212 positioned perpendicularly to the longitudinal axis of device 200. Unlike in device 10, where the opening of gap 12 is parallel to the longitudinal axis of device 10, the opening of gap 212 is perpendicular to the longitudinal axis of device 200.

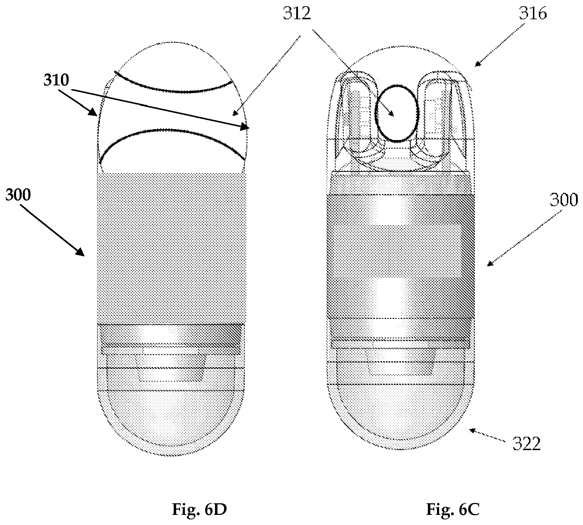

Reference is now made to Figs. 6C-6D which illustrate front and side views, respectively, of another imaging in-vivo diagnostic device, in accordance with the present invention. In some embodiments, device 300 may comprise an imaging head 322 located opposite a sensing head 316, whereas in other embodiments, device 300 need not comprise an imaging head 322 in addition to sensing head 316. Device 300, shown in Figs. 6C-6D may comprise one tunnel 312 that passes throughout the entire sensing head 316. Device 300 may comprise a tunnel 312 instead of a gap (e.g., gap 12 of device 10). In some embodiments, as shown in Fig. 6D, tunnel 312 may comprise openings 310 of a diameter larger than the diameter of the tunnel 312 located between its openings 310. Openings of a diameter larger than the diameter of the middle/inside of the tunnel 312 may enable better flow of bodily fluids through tunnel 312, thus avoiding blockage between LEDs 13 and photodiode 14 and enabling proper operation of sensing head 316.

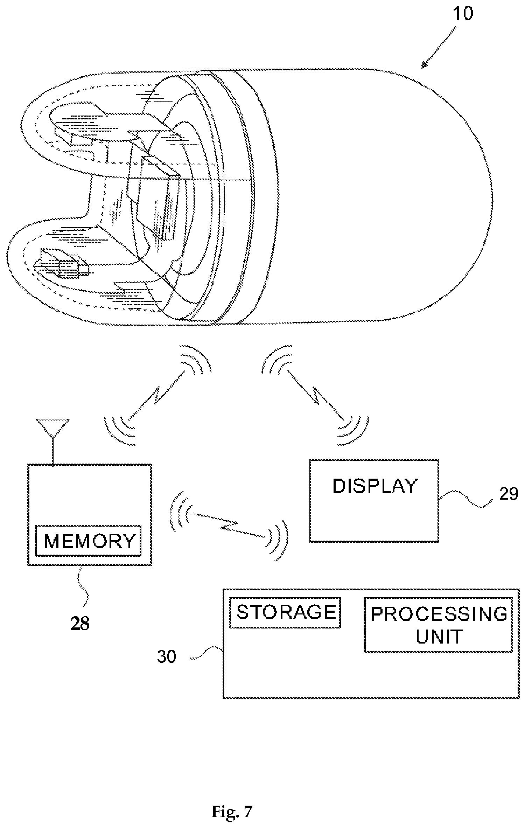

Reference is now made to Fig. 7, which presents a schematic illustration of a system for obtaining absorbance data, or the like, from an in-vivo diagnostic device, e.g., device 10, in accordance with embodiments of the present invention. The system comprises an in-vivo diagnostic device, e.g., device 10 (or any of devices 100, 200 or 300), external receiver/recorder 28, which is able to receive data (e.g., absorbance and/or image data) transmitted by the device 10, computing platform (workstation) 30, which may store, process, and analyze the received data, and optional display 29. Transmitter 20 transmits the signals, detected by photodiode 14, to external receiver 28, which may be portable, non-portable, mobile, non-mobile, wearable, or the like. In a particular embodiment, transmitter 20 may operate using radio waves. Other known wireless methods of transmission may be used. Transmitter 20 may include, for example, a transmitter module or sub-unit and a receiver module or sub-unit, or an integrated transceiver or transmitter-receiver.

Transmitter 20 may also be capable of receiving signals/commands, for example from an external transceiver. For example, transmitter 20 may include an ultra low power Radio Frequency (RF) high bandwidth transmitter, possibly provided in Chip Scale Package (CSP). Transmitter 20 may include different control capabilities, for example, for controlling the various operations of the device, although control capabilities or one or more aspects of control may be included in a separate component. Transmitter 20 is typically part of an ASIC (application specific integrated circuit), but may be of other constructions; for example, transmitter 20 may be a processor executing instructions. Transmitter 20 may transmit at any frequency selected from Industrial, Scientific and Medical radio frequencies (ISM). For example, when transmitter 20 is to transmit data related to signals detected by photodiode 14 alone, it may transmit at a frequency of 433.9Mhz, at bandwidth +/- 200Khz. And when, for example, transmitter 20 is to transmit image data as well as signals detected by photodiode 14, it may transmit at a frequency of 434.1Mhz, at bandwidth +/- 8Mhz.

In one embodiment, transmitter 20 may transmit data to receiver 28 via antenna 21, shown on Fig. 5B. While or after the in-vivo diagnostic device 10 passes through the GI tract, the signals detected by photodiode 14 may be transmitted by a transmitter 20 to external receiver 28, outside the patient's body. In a particular embodiment, receiver 28 may be a disposable receiver or a wearable disposable patch. It may possibly be close to or worn on a patient. A patient may wear the receiver and may swallow a new in-vivo diagnostic device every day for a week, for example, in order to constantly monitor the bodily fluid inside the GI tract, and consequently, detect the bleeding and determine the bleeding dynamics over time. Therefore, there may be a need to monitor the GI tract during a long period of time, e.g., a week, by inserting into a patient a new device every day during an examination period. The receiver 28 may include a visual indication, which may show where along the GI tract the bleeding was detected.

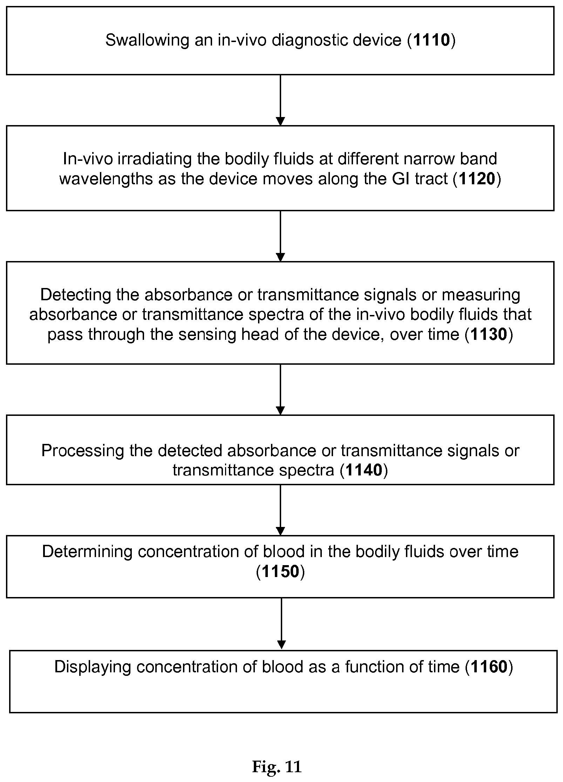

Receiver 28 may include a processor which may create absorbance spectra of the bodily fluids according to the signals detected from the at least four wavelengths. The processor may further compare the absorbance spectra of the in-vivo bodily fluids to a reference absorbance spectra of bile and chlorophyll and to a reference absorbance spectra of blood, which are created by detecting absorbance spectra of bile, of chlorophyll and of blood in water and of different concentrations of chlorophyll vs. blood, and bile vs. blood, and thus determine whether there is chlorophyll in-vivo, whether there is bile in-vivo, and whether there is blood or whether there is all three. Furthermore, the processor may compare between the measured absorbance spectra with the reference spectra and may determine the concentration of either bile, blood or both. When there is all bile, chlorophyll and blood present in the same sample, the processor, by comparing the measured absorbance spectra to the reference spectra, may indicate whether the ratios between the bile or the chlorophyll and blood indicate bleeding or whether the results indicate high concentration of blood but with no real bleeding, which may also indicate a pathology. In other embodiments, the concentration of blood, along with other detected in-vivo data, may indicate the location of blood in-vivo. In other embodiments, instead of comparing between transmittance or absorbance spectra, a comparison between discrete signals detected by photodiode 14 and a predetermined threshold may be done, as described with respect to Figs. 11-12 of WO 2010/086859, mentioned above.

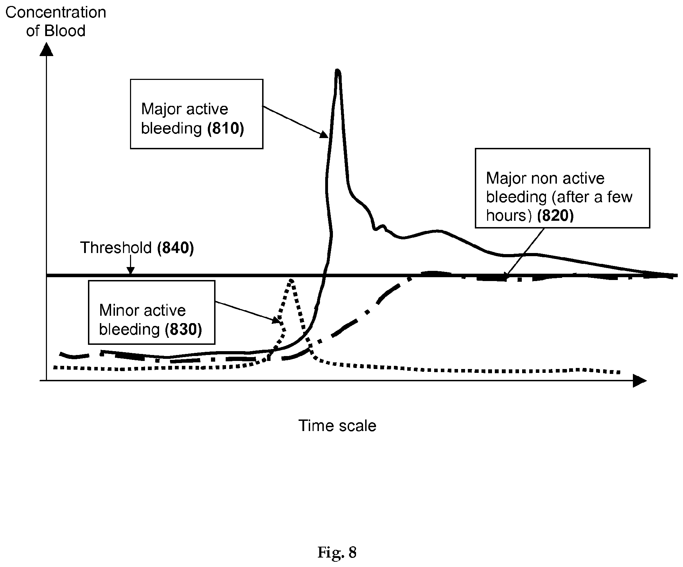

In still further embodiments, receiver 28 may comprise a memory unit for storing the data transmitted from the in vivo device over time. Receiver 28 may include one or more antenna elements or arrays, for example, in order to improve signal reception and/or to allow localization of the in-vivo device. The in-vivo device may optionally include a processing unit separately from transmitter 20 that may, for example, contain or process instructions. In other embodiments, instead of receiver 28 comprising a memory unit, the device (e.g. device 10, 100, 200 or 300) may comprise a memory unit for recording and storing the detected signals/data. The device may then transmit the data to a processor for processing the data to calculate level of concentration of blood over time (as will be described below in Fig. 8) and a display system may then display it to a user.

In some embodiments, the receiver 28 (or the device in itself) may be operatively associated with a computing platform or workstation 30, which may, for example, store the received data (e.g., image data and/or other data), process the received data (e.g., using a processor), store the data in a storage unit, display the received data and/or processed data (e.g., using a monitor), analyze the data, perform post-processing operations, perform real-time processing operations, or the like.

In some embodiments, the in-vivo diagnostic device may communicate with an external receiving and display system 29 (e.g., monitor) to provide display of data, for example, the absorbance spectra or a single optical density value, control, or other functions. In some embodiments, the display may be a separate unit not part of the computing device 30. In other embodiments, display 29 may display the absorbance spectra along with other information, such as pH values at the correlating in-vivo locations of where the pathological lesions are detected or expected. In other embodiments, where the device may for example comprise an imager and a broad band illumination, i.e., white light, in-vivo images may be displayed either alone or alongside the in-vivo locations where blood, or bile, or chlorophyll, or all them are detected. Other embodiments may have other configurations and capabilities. For example, components may be distributed over multiple sites or units, and control information or other information may be received from an external source.

According to some embodiments, the in-vivo diagnostic may comprise a pH detector (not shown). An example for a pH detector may be the pH detector by Endonetics Inc. as disclosed in U.S. Patent No. 6,689,056. Such a pH detector may continuously detect pH levels, and transmitter 20 may transmit the detected pH values along with the signals detected by photodiode 14 to a receiver external to a patient's body. Since, at different locations along the GI tract there are substantially different pH levels, the detected pH may indicate the in-vivo location. For example, in the stomach there is a low acidic pH of between 1 to 4, while in the small bowel the pH values are between 7 to 8 (slightly alkaline), and in the colon the pH is between 5.5 and 7 (slightly acidic).

In other embodiments, the in-vivo location where bleeding is detected may be calculated by an algorithm, such as disclosed in U.S. Patent No. 7,596,403 or by other algorithms. U.S. Patent No. 7,596,403 discloses a method for determining path length through a body lumen, for example, path length or distance to a specified location. This information may be used alone or in combination with other in-vivo data, such as pH, in order to determine the in-vivo location in which light detector 14 detects light signals, which may indicate the presence of blood.

According to Fig. 2, and as mentioned above, six LEDs 13 may irradiate at 557, 574, 609, 626, 653 and 747nm. The ratio of absorbance of chlorophyll between intensity of detected light that is irradiated from at least two LEDs irradiating at wavelengths selected from 600nm to 750nm is typically very high, which is similar to how the absorbance spectrum of bile behaves. Therefore, in order to determine whether chlorophyll is present in-vivo at a specific location, there is a need for a calculation of a ratio between intensity of detected light that is irradiated from at least two LEDs irradiating at wavelengths selected from 600nm to 750nm, but which would give a result unique to the absorbance spectrum of chlorophyll and not to that of bile. For example, the ratio of absorbance between intensity of detected light resulting from one LED irradiating at a wavelength of 626nm and a second LED irradiating light at a wavelength of 747nm is calculated in that location. This ratio between intensity of detected light resulting from LEDs irradiating at wavelengths of 626nm and 747nm is then compared to a reference value, which may be calculated from the absorbance spectrum of chlorophyll in water, and may indicate the presence of chlorophyll. The ratio between intensity of detected light at 626nm and at 747nm in chlorophyll is typically larger than that ratio in bile; therefore this ratio is suitable to indicate the difference between the two. If the ratio calculated from in-vivo signals exceeds a certain threshold calculated by the processor from the reference spectra, it may be an indication to the presence of chlorophyll in-vivo in that specific location where the ratio between 626nm and 747nm was calculated.

In order to determine the presence of blood in-vivo, an additional ratio should be calculated and compared to a reference. The ratio of absorbance between intensity of detected light that is irradiated from at least two LEDs irradiating at wavelengths selected from 400nm to 600nm may be calculated. The ratio is then compared to a reference value which may be calculated from the absorbance spectrum of blood in water. If the ratio calculated from the detected signals exceeds a certain threshold calculated based on the reference absorbance spectra of blood, this may indicate the presence of blood, which may indicate a pathology in-vivo.

In order to determine the presence of blood in-vivo and/or the presence of bile, LEDs 13 irradiate in at least three different narrow band wavelengths, among them, at least a first LED irradiating at a wavelength selected between 400nm to 600nm, at least a second LED irradiating at a wavelength selected between 600nm to 700nm, and at least a third LED irradiating at a wavelength selected between 700nm to 900nm. For example, LEDs 13 may comprise LEDs that irradiate at 576nm, 700nm and 850nm. In other embodiments, other numbers of LEDs may be used. For example, LEDs 13 may comprise six LEDs illuminating at wavelengths of: 557, 574, 609, 626, 653 and 747nm. Typically, a certain wavelength would be used in calculations of all of the ratios. This wavelength should be one that experiences good absorbance in blood, e.g., 747nm.

Reference is now made to Fig. 8 which is a graph describing different bleeding profiles over time, in accordance with embodiments of the present invention. A bleeding profile over time may be presented as a time line of a bleeding event, as shown in Fig. 8. In some embodiments, display of a bleeding profile over time may be a display of blood concentration (e.g., liter of blood per liter of in-vivo bodily fluids) as a function of time. Display of a bleeding event or a bleeding profile over time, which is based on analyzed data acquired by a sensing head, e.g., sensing head 16, may be a more efficient tool for determining presence of blood in-vivo (as well as determining the type of bleeding profile) compared to detection of redness levels in image data. A red area in an image does not necessarily indicate presence of bleeding but may, for example, appear due to a high concentration of blood vessels within the tissue. Whereas, analyzing data related to light absorbance of particles or fluids flowing within in-vivo bodily fluids and thus determining concentration of blood (over time) may provide a more accurate indication on presence of bleeding in-vivo, substantially eliminating errors that may occur due to the tissue background.

According to some embodiments, there may be a few different bleeding events or bleeding profiles in-vivo, which may correlate to different in-vivo pathologies. One of those bleeding events or profiles may be of "No Bleeding" (not shown in Fig. 8). Healthy patients should normally experience no bleeding along the GI tract. Therefore, in healthy patients the in-vivo diagnostic device 10 may detect no bleeding, e.g., the absorbance or transmittance signals detected by device 10 may not exceed a predetermined threshold, which is calculated based on the reference absorbance spectra of blood.

In some embodiments, the in-vivo diagnostic device may comprise an imaging head 22 in addition to the sensing head 16 that comprises gap 12 (e.g., Fig. 4A). In such devices it may be possible to shorten viewing times by initially viewing the analyzed data acquired by the sensing head 16, e.g., viewing the bleeding profile first and only then, if necessary, continue to view the image data acquired by the imaging head 22. In an alternative embodiment, the analyzed data acquired by the sensing head 16 may be displayed alongside the display of images captured by imaging head 22. The viewer of the image stream may use the analyzed data acquired by the sensing head 16 to identify portions of the image stream where a lesion or blood may be seen.

Another bleeding profile may be of "Major Active Bleeding" (810). For example, if the in-vivo diagnostic device passes along the small bowel just about the same time when there is major bleeding, a very high peak in blood concentration may be detected. After that peak, the farther the capsule is from that bleeding area, the lower the concentration of blood detected by device 10. In some embodiments, when a patient suffers from major bleeding, the computing device 30 or display system 29 may alert the physician and/or display the data related to the bleeding in a way the physician may not ignore, so as to ensure a quick assessment of the patient's condition by the physician. In some embodiments, when the in-vivo diagnostic device 10 comprises an imaging head 22, in addition to the sensing head 16 (e.g., Fig. 4 A), the analyzed data acquired by the sensing head 16 may be displayed alongside the display of a stream of images captured by imaging head 22.

A further bleeding profile or event may be of "Major Non-Active Bleeding" (820). In some patients, an ulcer, for example, may not bleed continuously. Instead, the ulcer may bleed for a first period of time after which it may cease bleeding for a second period of time, and then begin bleeding again, and so on. In some embodiments, if the in-vivo diagnostic device 10 reaches such an ulcer during its non-bleeding period, e.g., a few hours after a bleeding period, the peak of blood concentration may not be a high peak but rather a low and yet steady peak. The peak may be steady due to relatively long periods of bleeding, thus blood may be present along a large segment of the GI tract for a long time period, before it is less noticeable. For a physician to locate the area of the bleeding ulcer along the GI tract, he should refer to an area along the bleeding profile that is prior the steady peak. In some embodiments, the in-vivo device 10 may comprise an imaging head 22 in addition to the sensing head 16 (e.g., Fig. 4A). The analyzed data acquired by the sensing head 16 may then be displayed alongside the display of a stream of images captured by imaging head 22. Images that correspond to the area along the bleeding profile prior to the steady peak may be viewed in order to determine the in-vivo location of the bleeding ulcer.

Another bleeding profile or event may be of "Minor Active Bleeding" (730). Some patients may suffer from minor bleeding in the GI tract caused either by small ulcers or other in-vivo pathologies that are typically not harmful. Such minor or insignificant pathologies may appear as a distinct peak in blood concentration; however, since this is not a major bleeding, the peak may be a low peak.

In order to determine what is considered a high peak in blood concentration (thus indicating major bleeding), and what is considered a low peak in blood concentration (thus indicating minor bleeding), there is defined a threshold value (840) to which any bleeding profile may be compared. As an example, a blood concentration of 10⁻³ [liter of blood/liter of fluids] is considered to indicate a pathology. The calculated blood concentration over time may be compared with a predetermined threshold (840), e.g., 10⁻³ [liter of blood/liter of fluids] in order to determine whether the bleeding profile comprises blood concentration of above, equal to, or below 10⁻³ [liter of blood/liter of fluids], i.e., whether the bleeding profile indicates major bleeding, major non-active bleeding or minor bleeding, respectively. Other thresholds may be used.

According to some embodiments, in order to display blood concentration over time, the detected light signals should be analyzed and converted into blood concentration values. This conversion of light intensity to concentration may be done as described with respect to Figs. 11-12 in WO 2010/086859, mentioned above. In particular, the predetermined threshold (840) may be calculated, for example, from in-vitro experiments during which light intensity at different wavelengths is detected for bile in water of various concentrations, (and, in some embodiments, light intensity at different wavelengths is detected for chlorophyll in water of various concentrations), and light intensity at different wavelengths is detected for blood in water of various concentrations. As can be seen from Fig. 11 of WO 2010/086859, when the illuminated fluid contains bile and blood (spectra 111), the presence of bile causes the blood in water spectra (110BD) to be of a lower slope, i.e. the substantially high specificity of absorption by blood (110BD) of light at wavelengths between 400-600nm is not as high when in the presence of bile. Therefore, the predetermined threshold 113, or in this invention threshold (840), is an increasing threshold; the higher the bile concentration, the higher the threshold is. Other methods may be used in order to convert the detected light signals to concentration of blood.

According to some embodiments, the display of bleeding profiles to the physician may be similar to the graph in Fig. 8, e.g., it may include the threshold value (840) and values of blood concentration as detected by the in-vivo device, e.g., device 10, over time. In some embodiments, the display may comprise a graph of blood concentration over time, without the threshold value (840). In other embodiments, the bleeding profile may be presented alongside corresponding in-vivo images. In yet other embodiments, the bleeding profile graph may be interactive such that a click on the time scale (or on any location along the graph of the bleeding profile) may refer the physician to a corresponding in-vivo image. Other methods of display may be used.

In order to enable display of data related to bleeding profile over time, the in-vivo diagnostic system, e.g., the external receiver 28, comprises a memory unit. The data collected over time by the sensing head (and in some embodiments, the imaging data) is stored in the memory unit which may be located in external receiver 28. A memory unit may additionally be provided in the in-vivo device, e.g., device 10. The stored data may then be processed and analyzed by, for example, computing platform 30, which may be operatively associated with the receiver 28 or the device 10. The processed data may then be presented/displayed to the physician so the physician can make a quick and precise diagnosis of the patient's condition.

In some embodiments, the external receiver 28 comprises the memory unit, the processing unit and a display, such that there is no need to transfer the collected data to an additional computing platform 30. In these embodiments the entire system may comprise the in-vivo diagnostic device 10 and the external receiver 28 alone, which may make it easier on a patient to undergo the majority if not the entirety of the procedure of blood detection in a home setting instead of a hospital setting.

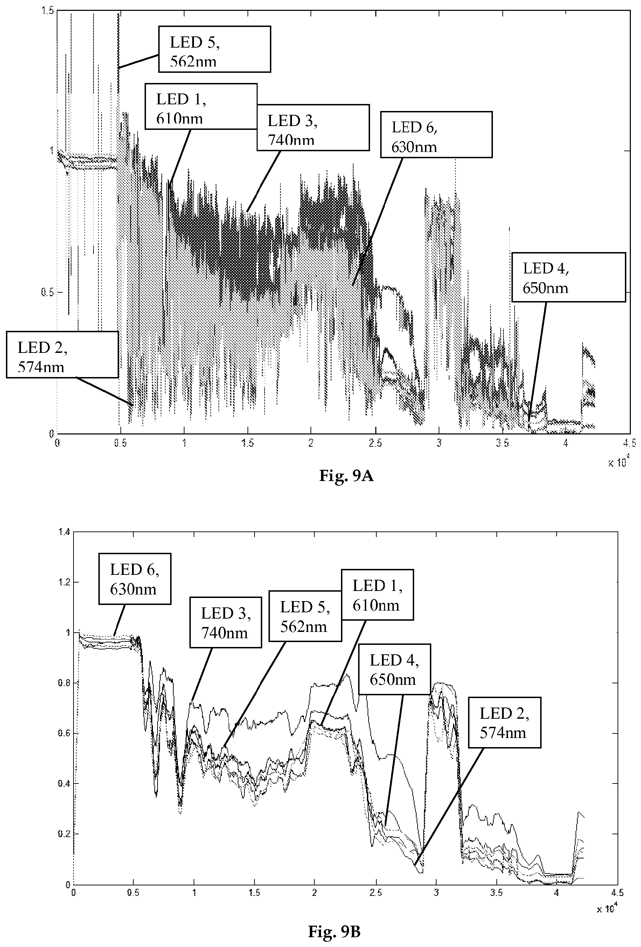

Reference is now made to Figs. 9A-9B, which are graphs showing detected light signals as a function of time before and after processing, respectively. According to some embodiments, processing of the acquired data over time and display of the processed data over time may be important for a few reasons. The detection of light signals by a sensing head, e.g., sensing head 16, may be affected by noises, as shown in Fig. 9A. Noises in the detected signals may occur due to debris, floaters or air bubbles present in the in-vivo bodily fluids that pass through gap 12, or due to tissue getting stuck within gap 12. For example, since in the small bowel the tissue wall may be tightly pressed against the diagnostic device (e.g., device 10), specifically during contractions occurring as part of peristaltic movement of the GI tract, the tissue wall may be pushed into gap 12. Other noises may appear due to high turbulences or increased flow of fluids during contractions, thus causing the fluid to be cloudier, all of which may affect the illumination conditions of LEDs 13, thereby affecting the readings of photodiode 14. Therefore, when the system comprises a memory unit (for example as part of the receiver, and optionally also as part of the diagnostic device) and detected data is saved as a function of time, various filters may be applied onto the data in order to clean, smooth and substantially decrease the noises, as shown in Fig. 9B. For example, various averaging filters may be used to clean noises, e.g., a moving average filter as implemented on the signals shown in Fig. 9A resulting with clean signals shown in Fig. 9B. In other embodiments, other filters and processing methods may be used.

Following processing of the acquired light signals over time in order to substantially reduce noise and display of the "clean" data, one may identify passage of the diagnostic device (e.g., device 10) from one area in the GI tract to another. For example, since the stomach comprises a different composition of fluids than the composition of fluids present in the small bowel, the detected intensity of light may be different in the stomach compared to the detected intensity of light in the small bowel. Equivalently, since the composition of fluids in the small bowel is different than the composition of fluids present in the colon, there should be a clear difference between detected light intensities of the two GI areas, namely the small bowel and the colon. In some embodiments, since without a special cleaning or preparation method the colon is typically filled with fluids that are cloudier than the fluids flowing in the small bowel, the intensity of transmission spectra in the colon is lower than in the small bowel (i.e., absorption of light in the colon is higher than in the small bowel).

By displaying the data over time, it may be easier to determine a change in intensity of transmission (or absorption) which may indicate a change in location of the diagnostic device along the GI tract, e.g., passage of the in-vivo diagnostic device from one area in the GI tract to another. In some embodiments, determining a change in GI tract area (based on change in intensity of transmission spectra) may be done by a physician observing the displayed data. Yet, according to other embodiments, determining a change in location of the in-vivo diagnostic device (e.g., device 10) along the GI tract may be done automatically by a processor, using various algorithms applied onto the "clean" data over time.

When the detected data is further processed such that intensity of detected light is converted to blood concentration (and not only "cleaned" from noises), display of blood concentration as a function of time may indicate the type of bleeding profile, thereby indicating patient condition, as described above (Fig. 8).

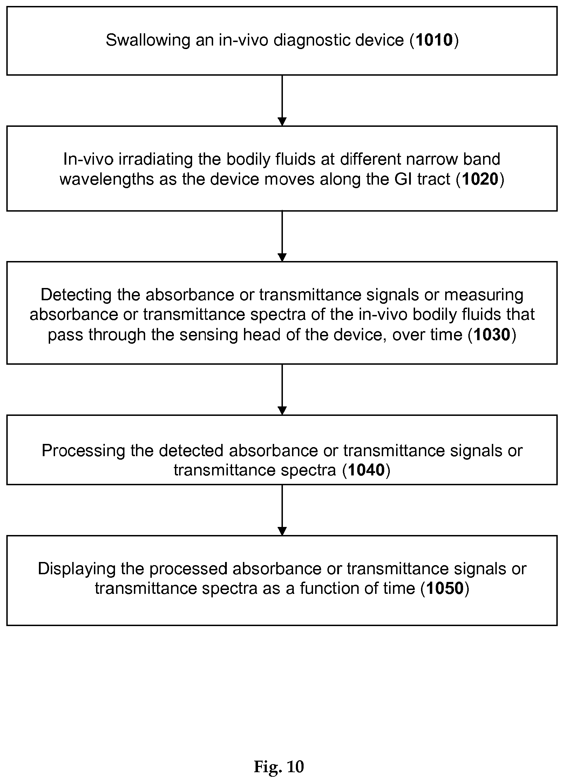

Reference is now made to Fig. 10 which shows a flow chart describing a method of using an in-vivo diagnostic device for detection of bleeding in the GI tract. The in-vivo diagnostic method may include the following steps:
(i) swallowing an in-vivo diagnostic device (1010);
(ii) in-vivo irradiating the bodily fluids at different narrow band wavelengths as the device moves along the GI tract (1020);
(iii) detecting the absorbance or transmittance signals or measuring absorbance or transmittance spectra of the in-vivo bodily fluids that pass through the sensing head of the device, over time (1030);
(iv) processing the detected absorbance or transmittance signals or transmittance spectra (1040); and
(v) displaying the processed absorbance or transmittance signals or transmittance spectra as a function of time (1050).

The method may additionally comprise comparing the processed absorbance or transmittance signals or transmittance spectra with a predetermined threshold prior to displaying the processed data. The method may further comprise comparing the absorbance or transmittance spectra of the in-vivo bodily fluids to a predetermined absorbance or transmittance spectra of bile and chlorophyll and determining the presence and concentration of bile and chlorophyll in the bodily fluids. The method may further comprise the step of determining concentration of blood in the bodily fluids over time, following the step of processing the detected absorbance or transmittance signals or spectra over time. The method may comprise the step of acquiring additional data other than absorbance or transmittance signals or transmittance spectra, transmitting the acquired additional data, and analyzing the additional data. The additional data that may be acquired by an in-vivo diagnostic device, such as device 10, may be in-vivo images, pH or any other in-vivo related data.

The step of displaying the processed absorbance or transmittance signals or transmittance spectra as a function of time (1050) may comprise displaying the concentration of blood as a function of time. The step of displaying (1050) may comprise displaying concentration of blood, alongside displaying a video of in-vivo images stream. The in-vivo images that may be displayed alongside the blood concentration data are images that are acquired at substantially the same time as the detection of absorbance or transmittance signals or measurement of absorbance or transmittance spectra of the in-vivo bodily fluids.

The step of processing the detected absorbance or transmittance signals or transmittance spectra (1040) may comprise filtering of the detected signals or spectra in order to "clean" the signals or spectra by reducing noises. The step of displaying the processed absorbance or transmittance signals or transmittance spectra as a function of time (1050) may comprise displaying the "cleaned" absorbance or transmittance signals or transmittance spectra over time. Following the step of displaying (1050), the method may further comprise the step of determining passage of the in-vivo diagnostic device from one area in the GI tract to another. Determining passage from one area to another along the GI tract may be done either by a physician or by a processor that may be part of receiver 28, computing platform 30, or even the diagnostic device 10.

The method may comprise the step of transmitting the detected absorbance or transmittance signals or transmittance spectra of the in-vivo bodily fluids with a transmitter, e.g., transmitter 20 to an external receiver, e.g., receiver 28 or to a computing platform, e.g., computing platform 30, prior to the step of processing the detected signals or spectra

(1040). The method need not comprise the step of transmitting the detected signals as the diagnostic device (e.g., device 10) may perform the processing step by itself. In such case, the method may comprise the step of transmitting the processed signals to a display system prior to the step of displaying the processed signals or spectra (1050).

Reference is now made to Fig. 11, which shows a flow chart describing a method of using an in-vivo diagnostic device for detection of bleeding in the GI tract. The in-vivo diagnostic method may include the following steps:
(i) swallowing an in-vivo diagnostic device (1110);
(ii) in-vivo irradiating the bodily fluids at different narrow band wavelengths as the device moves along the GI tract (1120);
(iii) detecting the absorbance or transmittance signals or measuring absorbance or transmittance spectra of the in-vivo bodily fluids that pass through the sensing head of the device, over time (1130);
(iv) processing the detected absorbance or transmittance signals or transmittance spectra (1140);
(v) determining blood concentration in the bodily fluids over time (1150); and
(vi) displaying blood concentration as a function of time (1160).

The step of determining blood concentration (1150) may comprise comparing the processed absorbance or transmittance signals or transmittance spectra with a predetermined threshold. The method may further comprise comparing the absorbance or transmittance signals or transmittance spectra of the in-vivo bodily fluids to a predetermined absorbance or transmittance spectra of bile and chlorophyll and determining the presence and concentration of bile and chlorophyll in the bodily fluids, prior to the step of displaying (1160).

The method may comprise the step of transmitting the detected absorbance or transmittance signals or transmittance spectra of the in-vivo bodily fluids with a transmitter, e.g., transmitter 20 to an external receiver, e.g., receiver 28 or to a computing platform, e.g., computing platform 30, prior to the step of processing the detected signals or spectra (1140). The method need not comprise the step of transmitting the detected signals as the diagnostic device (e.g., device 10) may perform the processing step by itself. In such case, the method may comprise the step of transmitting the processed signals to a display system prior to the step of displaying blood concentration over time (1160).

The step of displaying blood concentration as a function of time (1160) may comprise displaying concentration of blood, alongside displaying a video of in-vivo images stream. The in-vivo images that may be displayed alongside the blood concentration data are images that are acquired at substantially the same time as the detection of absorbance or transmittance signals or measurement of absorbance or transmittance spectra of the in-vivo bodily fluids.

The method may comprise the step of acquiring additional data other than absorbance or transmittance signals or transmittance spectra, transmitting the acquired additional data, and analyzing the additional data. The additional data that may be acquired by an in-vivo diagnostic device, such as device 10, may be in-vivo images, pH or any other in-vivo related data.

According to some embodiments, in order to ensure that an in-vivo diagnostic device, e.g., device 10, provides reliable results, device 10 may comprise two LEDs 13 of the same wavelength for each of the three ranges from which LEDs 13 may be selected. As described above, LEDs 13 are selected from three ranges: 400-600nm, 600-700nm and 700-900nm. For example, two LEDs may irradiate at 557nm (selected from the first range of 400-600nm), two LEDs may irradiate at 626nm (selected from the second range of 600-700nm) and two LEDs may irradiate at 747nm (selected from the third range of 700-900nm). When using a pair of similar LEDs 13, one LED of the pair may be used as a reference to the other LED. According to some embodiments, absorbance or transmittance signals of each of every two similar LEDs may be compared. In some embodiments, only when the signals of each LED of any pair are similar, are these signals processed. If the signals of each of any pair of LEDs are not similar, these signals are not processed, as the results are not reliable enough.

In other embodiments, since six LEDs 13 may constitute several combinations of three different LEDs, a processor may determine which set of three different LEDs is best to use. Based on the composition of in-vivo bodily fluids present in the GI tract, which mainly comprise blood, bile, and chlorophyll, intensity of absorbance of such fluids of irradiated illumination at wavelengths selected from the range of 400-600nm should be higher compared to intensity of absorbance of irradiated illumination at wavelengths selected from the range of 600-700nm. Furthermore, intensity of absorbance of GI fluids of illumination at wavelengths selected from the range of 600-700nm should be higher compared to intensity of absorbance of illumination at wavelengths selected from the range of 700-900nm. For example, intensity of absorbance of the GI tract fluids of light from an LED irradiating at 557nm should be higher than intensity of absorbance of light from an LED irradiating at 626nm, which should be higher than intensity of absorbance of light from an LED irradiating at 747nm. Other LEDs irradiating at different wavelengths may be used, as long as comparison between intensity of absorbance of light is done between LEDs from the different ranges mentioned above. A processor may select a set of three different LEDs from the total six LEDs, which comply with the mentioned order of intensity of absorbance levels (as described above regarding the three ranges of wavelengths) at a specific time period, and may process their corresponding detected signals at that specific time period. However, when there is no such order of intensity of absorbance in none of the combinations of three different LEDs at a specific time period, as described above regarding the three ranges of wavelengths, none of the signals detected at that specific time period is processed, since there is an indication that the results are not reliable.

In some embodiments, the in-vivo diagnostic device, e.g., device 10, may comprise an imaging head located on the device's end opposite gap 12. Image data acquired by such a device may be used in order to determine which signal may be considered as a bleeding event. An image acquired simultaneously with a detected signal may be used to determine whether the signal indicates presence of blood or not, which may assist in receiving reliable results of bleeding detection by the sensing head of device 10.

In some embodiments, when an in-vivo device, e.g., device 10, comprises an imaging head in addition to a sensing head (e.g., the imaging head may be positioned opposite the sensing head), the imaging head (e.g., imaging head 22) and sensing head (e.g., sensing head 16) need not operate simultaneously; instead each of the device's heads may operate alone during different time periods and/or at different in-vivo locations. For example, the sensing head may operate since swallowing the device and as long as device 10 is in the stomach. After a predetermined time period, which is longer than the time it typically takes an autonomous device to pass through the stomach, or after device 10 recognizes it is no longer in the stomach but rather in the small bowel (using any of known methods, e.g., change in pH), the imaging head may begin its operation, whereas the sensing head may cease its operation. After a further period of time, which is longer than the time it typically takes a device to pass through a normally configured small bowel, or after device 10 recognizes it is no longer in the small bowel but rather in the colon (using any of known methods, e.g., change in pH), the imaging head may cease its operation, whereas the sensing head may begin its operation again. In other embodiments, the sensing head and imaging head may operate simultaneously at certain in-vivo locations or certain time periods, while operating apart at other in-vivo locations or other time periods. Other combinations of operation of the sensing and imaging heads may be used.

The above methods according to Figs. 10-11 may further comprise acquiring an in-vivo image or obtaining other data such as pH value for localization of the device. In this case, the device may comprise an imager and white light illumination sources positioned on a different side of the device, e.g., opposite the end of the device containing a gap, as shown in Figs. 4A-4C, such that an in-vivo image may indicate the location in-vivo, along with the determination regarding the presence of blood provided by the sensing head. In other embodiments, the device may include a pH detector such that, based upon the pH level detected, a location of the device along the GI tract may be determined. In yet other embodiments, the localization of the device may be done using spectral information of bile and/or chlorophyll, while the spectral information of blood may indicate the presence of blood in-vivo, and in-vivo images may show the source of the bleeding on the tissue if any.

In some other embodiments, instead of comparing between absorbance spectra, the method may compare between the discrete signals detected by photodiode 14 and a predetermined threshold, as described with respect to Figs. 11-12 in WO 2010/086859, mentioned above.

Endoluminal bodily fluid may include, for example, tumor markers. Tumor markers may include molecules expressed in bodily fluid or tissue that are associated with cancer. Typically, tumor markers may be cancerous cells or products of cancerous cells, and may represent aberrant production of what may be a typically normal element. Some markers, such as antibodies, may be produced in response to the presence of cancer. Tumor marker targeted molecules may have a high affinity to tumor markers and, under certain conditions, may adhere to tumor markers in a liquid environment. These may include antigens having specificity to tumor marker antibodies. Alternatively, tumor marker targeted molecules may include antibodies specific to tumor marker antigens. A patient may swallow tumor marker targeted molecules in advance, before swallowing the in-vivo diagnostic device, such that while the device passes along the GI tract, it may detect the marked tissue. Bodily fluid samples may be analyzed for other chemicals, compounds or molecules.

Although portions of the discussion herein may relate to six LEDs for irradiating the bodily fluid, the present invention is not limited in this regard, and may include, for example, seven or more LEDs, or less than six LEDs.

A system in accordance with some embodiments of the invention may be used, for example, in conjunction with a device which may be inserted into a human body. However, the scope of the present invention is not limited in this regard. For example, some embodiments of the invention may be used in conjunction with a device which may be inserted into a non-human body or an animal body.

While certain features of the invention have been illustrated and described herein, many modifications, substitutions, changes, and equivalents will now occur to those of ordinary skill in the art.

## Claims

1. An in-vivo diagnostic system for the detection of bleeding inside the GI tract of a patient, said system comprising:
an in-vivo sensing device (10) comprising:
a housing (11) comprising a sensing head (16) in the form of a gap (12) which remains in contact with bodily fluids and through which the fluids pass when the device (10) is within the GI tract;
said sensing head (16) comprising:
at least three LEDs (13) positioned on a first side of the gap (12), each LED (13) for irradiating the fluids within the gap (12), wherein each LED (13) irradiates the fluids at a different narrow band wavelength, and wherein at least one LED of said at least three LEDs (13) irradiates at a wavelength selected from a range of 400nm to 600nm, at least one LED irradiates at a wavelength selected from a range of 600nm to 700nm, and at least one LED irradiates at a wavelength selected from a range of 700nm to 900nm;
at least one light detector photodiode (14) positioned on a second side of the gap (12) opposite to the first side and facing the LEDs (13), for detecting light from said LEDs (13) which passes through the fluids; and
a transmitter (20) for transmitting data collected from the at least one light detector photodiode (14) of the sensing head (16) of the in-vivo device (10);
the system further comprising, external to the in-vivo sensing device (10):
a receiver (28) for receiving the data transmitted by the transmitter (20);
a memory unit for storing the data received by the external receiver (28); and
a processing unit configured to process the stored data so as to determine a series of blood concentration values measured over time, and to identify a bleeding event based on the blood concentration values as a function of time and based on a comparison of the blood concentration values to a threshold.

2. The system according to claim 1, wherein the processing unit is configured to identify a bleeding event indicating major-active bleeding over time if the blood concentration values show a peak above the threshold value after which blood concentration values measured over time are below the peak but are still above the threshold value.

3. The system according to claim 1, wherein the processing unit is configured to identify a bleeding event indicating major-non-active bleeding over time if the blood concentration values show a steady peak close to the threshold value.

4. The system according to claim 1, wherein the processing is configured to identify a bleeding event indicating minor bleeding over time if the blood concentration values show at least one peak below the threshold value.

5. The system according to claim 1, wherein the processing is configured to identify a bleeding event indicating no bleeding over time if the blood concentration values are below the threshold value with no distinct peak over time.

6. The system according to claim 1, wherein said memory unit and said processing unit are incorporated within said external receiver (28).

7. The system according to claim 1, wherein said sensing head (16) comprises a cover (110) placed over the gap (12), said cover (110) comprising at least one hole (61) on each side of the gap (12).

8. The system according to claim 1, wherein said in-vivo sensing device (10) further comprises an imaging head (22).

9. The system according to claim 8, wherein said imaging head (22) is located at one end of said housing (11), opposite to the end of the housing (11) comprising the sensing head (16).

10. The system according to claim 1, wherein said system further comprises a display (29) for displaying a graph of the blood concentration values over time.

## Patentansprüche

1. In-vivo-Diagnosesystem zur Detektierung von Blutungen im Gastrointestinaltrakt eines Patienten, umfassend:
eine in-vivo-Sensorvorrichtung (10), umfassend:
ein Gehäuse (11), umfassend einen Sensorkopf (16) in Form eines Spalts (12), der mit Körperflüssigkeiten in Kontakt bleibt und durch den die Flüssigkeit hindurch gelangt, wenn sich die Vorrichtung (10) im GI-Trakt befindet;
wobei der Sensorkopf (16) umfasst:
mindestens drei LEDs (13), die auf einer ersten Seite des Spalts (12) positioniert sind, wobei jede LED (13) zum Bestrahlen der Flüssigkeiten innerhalb des Spalts (12) dient, wobei jede LED (13) die Flüssigkeiten mit einer anderen schmalbandigen Wellenlänge bestrahlt, und wobei mindestens eine LED der mindestens drei LEDs (13) mit einer Wellenlänge ausgewählt aus einem Bereich von 400 nm bis 600 nm bestrahlt, mindestens eine LED mit einer Wellenlänge ausgewählt aus einem Bereich von 600 nm bis 700 nm bestrahlt, und mindestens eine LED mit einer Wellenlänge ausgewählt aus einem Bereich von 700 nm bis 900 nm bestrahlt;
mindestens eine Lichtdetektor-Fotodiode (14), die auf einer zweiten Seite des Spalts (12) gegenüber der ersten Seite und zu den LEDs (13) weisend positioniert ist, um Licht von den LEDs (13) zu detektieren, welches durch die Flüssigkeiten hindurch gelangt; und
einen Sender (20) zur Übertragung von Daten, die von der mindestens einer Lichtdetektor-Fotodiode (14) des Sensorkopfes (16) der in-vivo-Vorrichtung (10) aufgefangen wurden;
wobei das System ferner außerhalb der in-vivo-Sensorvorrichtung (10) umfasst:
einen Empfänger (28) zum Empfangen der von dem Sender (20) übertragenen Daten;
eine Speichereinheit zum Speichern der vom externen Empfänger (28) empfangenen Daten; und
eine Verarbeitungseinheit, die ausgelegt ist, um die gespeicherten Daten zu verarbeiten, um eine Reihe von Blutkonzentrationswerten zu bestimmen, die im Zeitverlauf gemessen werden, und um ein Blutungsereignis anhand der Blutkonzentrationswerte als Funktion der Zeit und basierend auf einem Vergleichs der Blutkonzentrationswerte mit einem Schwellenwert zu identifizieren.

2. System nach Anspruch 1, wobei die Verarbeitungseinheit dazu ausgelegt ist, ein Blutungsereignis zu identifizieren, das eine größere aktive Blutung im Zeitverlauf angibt, wenn die Blutkonzentrationswerte einen Peak oberhalb des Schwellenwerts zeigen, nach dem die im Zeitverlauf gemessenen Blutkonzentrationswerte unterhalb des Peaks liegen, aber nach wie vor oberhalb des Schwellenwerts liegen.

3. System nach Anspruch 1, wobei die Verarbeitungseinheit dazu ausgelegt ist, ein Blutungsereignis zu identifizieren, das eine größere inaktive Blutung im Zeitverlauf angibt, wenn die Blutkonzentrationswerte einen stationären Peak nahe dem Schwellenwert zeigen.

4. System nach Anspruch 1, wobei die Verarbeitung dazu ausgelegt ist, ein Blutungsereignis zu identifizieren, das geringfügige Blutung im Zeitverlauf angibt, wenn die Blutkonzentrationswerte mindestens einen Peak unterhalb des Schwellenwerts zeigen.

5. System nach Anspruch 1, wobei die Verarbeitung dazu ausgelegt ist, ein Blutungsereignis zu identifizieren, das keine Blutung im Zeitverlauf angibt, wenn die Blutkonzentrationswerte unterhalb des Schwellenwerts liegen, ohne dass ein eindeutiger Peak im Zeitverlauf vorliegt.

6. System nach Anspruch 1, wobei die Speichereinheit und die Verarbeitungseinheit in den externen Empfänger (28) integriert sind.

7. System nach Anspruch 1, wobei der Sensorkopf (16) eine Abdeckung (110) umfasst, die über dem Spalt (12) platziert ist, wobei die Abdeckung (110) mindestens ein Loch (61) auf jeder Seite des Spalts (12) umfasst.

8. System nach Anspruch 1, wobei die in-vivo-Sensorvorrichtung (10) ferner einen Bildgebungskopf (22) umfasst.

9. System nach Anspruch 8, wobei sich der Bildgebungskopf (22) an einem Ende des Gehäuses (11) gegenüber von dem Ende des Gehäuses (11) befindet, welches den Abtastkopf (16) umfasst.

10. System nach Anspruch 1, wobei das System ferner eine Anzeige (29) zum Anzeigen eines Graphen der Blutkonzentrationswerte im Zeitverlauf umfasst.

## Revendications

1. Système de diagnostic in vivo pour la détection de saignements à l'intérieur du tractus gastro-intestinal d'un patient, ledit système comprenant :
un dispositif de détection in vivo (10) comprenant :
un boîtier (11) comprenant une tête de détection (16) sous la forme d'un espace (12) qui reste en contact avec des liquides corporels et à travers lequel passent les liquides lorsque le dispositif (10) est à l'intérieur du tractus gastro-intestinal ;
ladite tête de détection (16) comprenant :
au moins trois DEL (13) positionnées sur un premier côté de l'espace (12), chaque DEL (13) étant destinée à exposer les liquides à l'intérieur de l'espace (12) à un rayonnement, chaque DEL (13) exposant les liquides à une longueur d'onde à bande étroite différente, et au moins une DEL desdites au moins trois DEL (13) exposant à une longueur d'onde choisie dans la plage de 400 nm à 600 nm, au moins une DEL exposant à une longueur d'onde choisie dans la plage de 600 nm à 700 nm, et au moins une DEL exposant à une longueur d'onde choisie dans la plage de 700 nm à 900 nm ;
au moins une photodiode de photodétecteur (14) positionnée sur un second côté de l'espace (12) en regard du premier côté et faisant face aux DEL (13), pour détecter la lumière provenant desdites DEL (13) qui traverse les liquides ; et
un émetteur (20) destiné à émettre des données collectées à partir de l'au moins une photodiode de photodétecteur (14) de la tête de détection (16) du dispositif in vivo (10) ;
le système comprenant en outre, à l'extérieur du dispositif de détection in vivo (10) :
un récepteur (28) destiné à recevoir les données émises par l'émetteur (20) ;
une unité de mémoire destinée à stocker les données reçues par le récepteur externe (28) ; et
une unité de traitement configurée pour traiter les données stockées de manière à déterminer une série de valeurs de concentration sanguine mesurées dans le temps, et pour identifier un événement de saignement sur la base des valeurs de concentration sanguine en fonction du temps et sur la base d'une comparaison des valeurs de concentration sanguine à un seuil.

2. Système selon la revendication 1, dans lequel l'unité de traitement est configurée pour identifier un événement de saignement indiquant un saignement actif majeur dans le temps si les valeurs de concentration sanguine présentent un pic au-dessus de la valeur de seuil après lequel les valeurs de concentration sanguine mesurées dans le temps sont inférieures au pic mais sont toujours au-dessus de la valeur de seuil.

3. Système selon la revendication 1, dans lequel l'unité de traitement est configurée pour identifier un événement de saignement indiquant un saignement non actif majeur dans le temps si les valeurs de concentration sanguine montrent un pic stable proche de la valeur de seuil.

4. Système selon la revendication 1, dans lequel le traitement est configuré pour identifier un événement de saignement indiquant un saignement mineur dans le temps si les valeurs de concentration sanguine montrent au moins un pic en dessous de la valeur de seuil.

5. Système selon la revendication 1, dans lequel le traitement est configuré pour identifier un événement de saignement indiquant l'absence de saignement dans le temps si les valeurs de concentration sanguine sont en dessous de la valeur de seuil sans pic distinct dans le temps.

6. Système selon la revendication 1, dans lequel ladite unité de mémoire et ladite unité de traitement sont incorporées à l'intérieur dudit récepteur externe (28).

7. Système selon la revendication 1, dans lequel ladite tête de détection (16) comprend un couvercle (110) placé sur l'espace (12), ledit couvercle (110) comprenant au moins un trou (61) de chaque côté de l'espace (12).

8. Système selon la revendication 1, dans lequel ledit dispositif de détection in vivo (10) comprend en outre une tête d'imagerie (22).

9. Système selon la revendication 8, dans lequel ladite tête d'imagerie (22) est située à une extrémité dudit logement (11), en regard de l'extrémité du logement (11) comprenant la tête de détection (16).

10. Système selon la revendication 1, dans lequel ledit système comprend en outre un afficheur (29) pour afficher un graphique des valeurs de concentration sanguine dans le temps.
